# EUROPEAN PATENT APPLICATION

(11) **EP 2 105 495 A1**
(43) Date of publication of application: **30.09.2009**
(21) Application number: 08153632.8
(22) Date of filing: 28.03.2008
(51) Int. Cl.: C12M 1/107, C12N 1/12

(54) **System for biowaste usage and production of energy and food/feed**

(71) Applicant: Friesland Brands B.V., 7943 PE Meppel (NL)
(72) Inventor: Bongers, Cornelis Margaretha Theodorus Maria, 5707 KR Helmond (NL); Bakker, Gijsbert Henk, 1461 AM Zuidoostbeemster (NL)
(74) Representative: Hatzmann, Martin

(57) **Abstract**

Disclosed is an integrated waste usage and energy production system comprising the following subsequent, connected units:
(a) an anaerobic digester unit;
(b) a liquid-solid separator unit
(c) a refinery unit
(d) an algae bioreactor unit
(e) an oil extraction unit

further comprising, in connection with both the digester unit (a) and the bioreactor unit (d) an electricity generating unit (f), i.e. a power plant, and optionally comprising a gasification unit (g) connected to the separator unit (b).

The system can be used in a process in which food or feed supplements from algae oil are made, together with energy production.

## Description

The invention pertains to the conversion of manure, particularly cattle manure, and, more particularly bovine manure, into useful end products. The invention also pertains to methods of obtaining biofuels and bioenergy as well as food and/or feed products.

Manure can be used as a biomass to be anaerobically digested so as to form biogas that can be put to use in *e.g.* a power plant. Reference is made, e.g. to an Integrated Bioenergy Center as developed by Sunflower Electric Power Corporation. A description thereof indicates that waste from livestock facilities will be processed through an anaerobic digester to extract methane which will be utilized to power an ethanol plant. An algae system will recover carbon dioxide from the power plant and nutrients from the livestock waste to create an oil source for the biodiesel facility and/or algae biomass for further processing into high value food- and/or feed products.

Algae provide food for people and livestock, serve as thickening agents in ice cream and shampoo and are used as drugs to ward off diseases. (Micro) algae can be used to enhance the nutritional value of food and animal feed owing to their chemical composition, which is high in essential proteins and unsaturated fats as well as in vitamins and mineral. Moreover, they are cultivated as a source of highly valuable molecules. Polyunsaturated fatty acid oils are added to infant formulas and algal pigments are important as natural dyes.

Micro-algae have been used in human nutrition for hundreds of years. Especially in Taiwan, Japan, Chile or Mexico, the micro-algae have been used as a source of protein and/or fat but also as a delicacy.

Small amounts of micro algae like Chlorella and Spirulina provide an exceptionally tremendous wealth of nutrients that are essential. They are thought to contain every nutrient required by the human body. Men could literally live off these micro- algae. If chlorella and spirulina were sold with Nutrit ion Facts labels, those labels would be impressive: zero refined carbohydrates, high in digestible protein, high in essential fatty acids, no bad fats, high in chlorophyll, and so on. Many analyses of gross chemical composition of different algae have been published in the literature. In order to give a general overview on the major constituents, selected data of various micro- algal species are compiled in table 1.

**Table 1: General composition of different algae (% of dry matter)**

| **Alga** | **Protein** | **Carbohydrates** | **Lipids** |
|---|---|---|---|
| *Anabaena cylindrical* | 43-56 | 25-30 | 4-7 |
| *Aphanizomenon flosaquae* | 62 | 23 | 3 |
| *Chlamydomonas rheinhardii* | 48 | 17 | 21 |
| *Chlorella pyrenoidosa* | 57 | 26 | 2 |
| *Chlorella vulgaris* | 51-58 | 12-17 | 14-22 |
| *Dunaliella salina* | 57 | 32 | 6 |
| *Euglena gracilis* | 39-61 | 14-18 | 14-20 |
| *Porphyridium cruentum* | 28-39 | 40-57 | 9-14 |
| *Scenedesmus obliquus* | 50-56 | 10-17 | 12-14 |
| *spirogyra* sp. | 6-20 | 33-64 | 11-21 |
| *Arthrospira maxima* | 60-71 | 13-16 | 6-7 |
| *Spirulina platensis* | 46-63 | 8-14 | 4-9 |
| *Synechococcus* sp. | 63 | 15 | 11 |

Throughout the world a number of bio-feedstock are currently being experimented for biodiesel (and ethanol) production. Herein algae have emerged as one of the most promising sources especially for biodiesel production, for two main reasons: the yields of oil from algae are orders of magnitude higher than those for traditional oilseeds, and algae can grow in places away from the farmlands and forests, thus minimising the damages caused to the eco- and food chain systems.

Despite many good theoretical possibilities to integrate waste usage, power generation, and fuel production, progress goes slowly in view of the relatively large investments to be made. Also, the regulatory environment in which these integrated systems operate have become more complex. Reference can be made to e.g. manure bookkeeping and to carbon dioxide reduction.

In view of the investments to be made, it were desired if a system in which manure (or other waste) usage and energy (power, gas) and especially food/feed production are integrated, could become more economical by a more complete turnover of the materials used. Also, it were desired to provide a process that is neutral or, preferably, negative on carbon dioxide generation.

In order to better address *inter alia* the foregoing objectives, the present invention, in one embodiment, provides an integrated bio waste (notably manure) usage and energy-, food and/or feed production system comprising the following subsequent, connected units:
(a) an anaerobic digester unit;
(b) a liquid-solid separator unit
(c) a refinery unit
(d) an algae bioreactor unit
(e) an oil extraction unit
further comprising, in connection with both the digester unit (a) and the bioreactor unit (d) an electricity generating unit (f), i.e. a power plant, and optionally comprising a gasification unit (g) connected to the separator unit (b).

The invention, in another embodiment, provides a method of converting biomass, particularly, manure into useful end products comprising the steps of:
(i) obtaining manure (*e.g.* from cattle such as cows);
(ii) subjecting the manure to an anaerobic digestion, e.g. by feeding it into the aforementioned digester unit (a);
(iii) obtaining gas output from the anaerobic digestion (mainly methane and carbon dioxide), and using this for energy production, *e.g*. by feeding the gas output into the electricity generating unit (f);
(iv) obtaining a non-gas output (*i.e*. a digestate) from the anaerobic digestion, and subjecting this to a separation of solids and liquids, *e.g.* in the liquid-solid separator unit (b);
(v) optionally, but preferably, subjecting the obtained solids to gasification, *e.g.* in the gasification unit (g), so as to obtain syngas (mainly a combination of H₂, CO, CO₂, and CH₄);
(vi) and using this gas for energy production, e.g. by feeding it into the electricity generating unit (f);
(vii) subjecting the obtained liquid to a refining step, *e.g*. by feeding it into the refinery unit (c), so as to obtain a refined (*e.g.* purified and/or microbiologically safe) liquid, and using this liquid to feed algae, *e.g*. in the algae bioreactor unit (d), so as to obtain output from algae bioreaction;
(viii) obtaining oxygen output from the algae bioreaction, and using this for energy production, *e.g*. by feeding the oxygen output into the electricity generating unit (f);
(ix) obtaining algae output from the algae bioreaction, and subjecting this to oil extraction, *e.g.* in the oil extraction unit (e);
(x) obtaining algae oil as the extract from the oil extraction, and putting this to use, *e.g.* in biodiesel,, in a feed supplement, or in a food supplement;
(xi) obtaining algae cake as the residue from the oil extraction, and putting this to use, *e.g.* in food applications by means of a biorefinery process and/or in the generation of bioethanol via carbohydrate fermentation.

As will be apparent from the description of the method, the present invention, and particularly the system described hereinabove, adds a useful component to biowaste (notably manure) usage and energy production, viz. the generation of further useful materials that can find application in energy, feed, and food. Without wishing to be bound by theory, the present inventors believe that the inclusion in the process of a refinery step, *i.e*. the inclusion in the system of the refinery unit (c), is pivotal to the added value that can be obtained from the present process.

The refinery step particularly refers to rendering the obtained materials microbiologically safe, as well as avoiding the presence of other factors inhibiting algae growth, such as pH, salts (notably potassium). Thus the refinery unit (c) preferably is a microbiological inactivation unit (*e.g*. sterilization, pasteurization, UHT, gamma irradiation, electron beam, UVC, ozone, HPP, filtration techniques (micro-, ultra-, nano-filtration, reversed osmosis) or a combination of these microbiological inactivation/separation methods in a cascade architecture).

The terms "connected" and "in connection with" should be broadly interpreted as referring to any way in which a connector allows output from one unit to be fed into the other unit, and/or *vice versa.* Although, preferably, such connectors are provided as physical means capable of transporting, as the case may be, gas or liquid actually attached to the units, *e.g.* a pipe or a pipeline, the connector can also be a less sophisticated means such as a truck or a train, if the units are not physically connected, or are even on distant sites from each other.

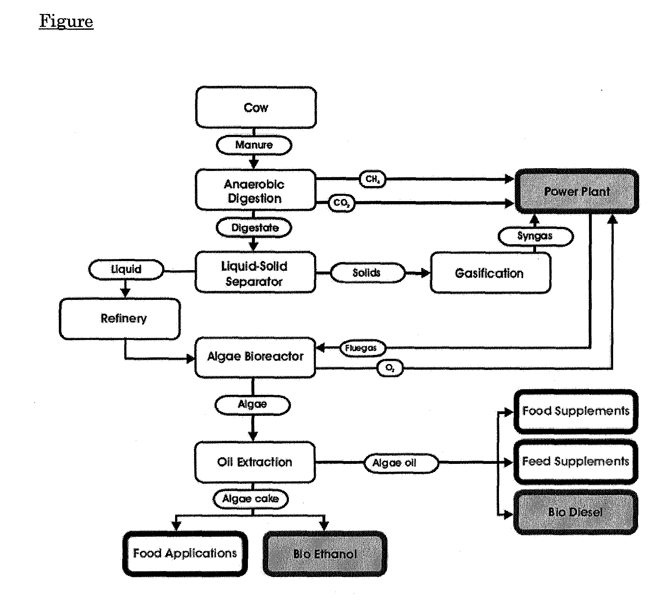

The Figure depicts the aforementioned units in a scheme, showing how they can be connected and which feeds (such as gas or liquid) are possibly fed into which unit.

The various process steps, as well as the system units that can be used therein, are known to the skilled person.

Anaerobic digesters are widely used, and do not require elucidation here. Nor does a liquid-solid separator (which is, *e.g.,* a centrifuge or a hydrocyclone), or an electricity generator (power plant). Refinery units are discussed above.

Algae bioreactors are e.g. photobioreactors used to harvest algae. A photobioreactor is basically a bioreactor that incorporates some type of light source. The term photobioreactor is more commonly used to define a closed system, as opposed to an open pond. A pond covered with a greenhouse could also be considered a photobioreactor. Because these systems are closed everything that the algae need to grow, (carbon dioxide, water and light) needs to be introduced into the system. Photobioreactors can be set up to be continually harvested (the majority of the larger cultivation systems), or by harvesting a batch at a time (like polyethlyene bag cultivation). A batch photobioreactor is set up with nutrients and algal seed, and allowed to grow until the batch is harvested. A continuous photobioreactor is harvested either continually, as daily, or more frequently. Some types of photobioreactors include: glass or plastic tubes, tanks,plastic sleeves or bags.

Algae oil extraction unit is known to the skilled person. This generally involves extracting the algae from their growth medium (using an appropriate separation process), and use the wet algae to extract the oil. (the algae need not be dried before oil extraction). There are three well-known methods to extract the oil from oilseeds, and these methods in principle apply equally well for algae: (1) expeller/press, (2) hexane solvent oil extraction, and (3) supercritical fluid extraction. As to the expeller/press method: when algae is dried it retains its oil content, which then can be "pressed" out with an oil press. Many commercial manufacturers of vegetable oil use a combination of mechanical pressing and chemical solvents in extracting oil. While more efficient processes are emerging, a simple process is to use a press to extract a large percentage (70-75%) of the oils out of algae. Algal oil can be extracted using chemicals. Benzene and ether have been used, but a popular chemical for solvent extraction is hexane, which is relatively inexpensive. Hexane solvent extraction can be used in isolation or it can be used along with the oil press/expeller method. After the oil has been extracted using an expeller, the remaining pulp can be mixed with cyclohexane to extract the remaining oil content. The oil dissolves in the cyclohexane, and the pulp is filtered out from the solution. The oil and cyclohexane are separated by means of distillation. These two stages (cold press & hexane solvent) together will be able to derived more than 95% of the total oil present in the algae. Supercritical Fluid Extraction can extract almost 100% of the oils all by itself. This method needs suitable equipment for containment and pressure, which equipment is known in the art. In the supercritical fluid/CO₂ extraction, CO₂ is liquefied under pressure and heated to the point that it has the properties of both a liquid and gas. This liquefied fluid then acts as the solvent in extracting the oil. Other extraction methods include: enzymatic extraction, which uses enzymes to degrade the cell walls with water acting as the solvent, this makes fractionation of the oil much easier; Osmotic shock, which is a sudden reduction in osmotic pressure that can cause cells in a solution to rupture. Osmotic shock is sometimes used to release cellular components, such as oil; Ultrasonic-assisted extraction, wherein ultrasonic waves are used to create cavitation bubbles in a solvent material, when these bubbles collapse near the cell walls, it creates shock waves and liquid jets that cause those cells walls to break and release their contents into the solvent.

Gasification is existing technology originally developed for the production of towngas from coal. During gasification a biomass material reacts with a small amount of air (not enough for complete combustion). In this way, fuel gas, a gas mixture of mainly CO and H2 is produced. After cleaning this combustible gas mixture can be used as fuel for gas engines, gas turbines or fuel cells with which (green) electricity is produced. The advantage of gasification against combustion is the fact that the cleaning the gas occurs at a much smaller gas volume. Also with gasification the overall efficiency of the conversion from biomass into electricity is higher. Many gasification systems have been developed for the gasification of wood, but facilities exist for the gasification of many different kinds of biomass matter. E.g. a circulating fluidized bed gasifier can be used.

## Claims

1. An integrated waste usage and energy-, food- and/or feed production system comprising the following subsequent, connected units:
(a) an anaerobic digester unit;
(b) a liquid-solid separator unit
(c) a refinery unit
(d) an algae bioreactor unit
(e) an oil extraction unit
further comprising, in connection with both the digester unit (a) and the bioreactor unit (d) an electricity generating unit (f), *i.e.* a power plant, and optionally comprising a gasification unit (g) connected to the separator unit (b).

2. A method of converting biomass, particularly, manure, into useful end-products comprising the steps of:
(i) obtaining manure;
(ii) subjecting the manure to an anaerobic digestion;
(iii) obtaining gas output from the anaerobic digestion (mainly methane and carbon dioxide), and using this for energy production;
(iv) obtaining a non-gas output (*i.e.* a digestate) from the anaerobic digestion, and subjecting this to a separation of solids and liquids,;
(v) optionally, but preferably, subjecting the obtained solids to gasification, so as to obtain syngas and using this gas for energy production;
(vi) subjecting the obtained liquid to a refining step, so as to obtain a refined liquid, and using this liquid to feed algae, so as to obtain food- and/or feedgrade output from algae bioreaction;
(vii) obtaining oxygen output from the algae bioreaction, and using this for energy production;
(viii) obtaining algae output from the algae bioreaction, and subjecting this to (food grade) oil extraction;
(ix) obtaining algae oil as the extract from the oil extraction, and putting this to use, *e.g*. in biodiesel,, in a feed supplement, or in a food supplement;
(x) obtaining algae cake as the residue from the oil extraction, and putting this to use, *e.g.* in food and/or feed applications or in the generation of bioethanol via carbohydrate fermentation.

3. A method according to claim 2, wherein one or more, and preferably all, steps are conducted in the respective units of the system as claimed in claim 1.

4. A method of manufacturing a food or feed supplement based on algae oil, wherein the algae oil is obtained in a method according to claim 2 or 3.
